# EUROPEAN PATENT APPLICATION

(11) **EP 4 588 918 A1**
(43) Date of publication of application: **23.07.2025**
(21) Application number: 24305113.3
(22) Date of filing: 18.01.2024
(51) Int. Cl.: C07D 471/04

(54) **SYNTHESIS OF (S)-1-(1-(3-CHLOROPHENYL)-2-(DIMETHYLAMINO)ETHYL)- 4-(5-MORPHOLINO-1H-PYRROLO[2,3-B]PYRIDIN-3-YL)PYRIDIN-2(1H)-ONE**

(71) Applicant: AGV Discovery, 34980 Montferrier-sur-Lez (FR)
(72) Inventor: BORIES, Cédric, 34830 Jacou (FR); MATHIEU, Loïc, 69003 Lyon (FR)
(74) Representative: Germain Maureau

(57) **Abstract**

The present invention concerns a chiral process for the preparation of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one.

## Description

### FIELD OF THE INVENTION

The present invention relates to a novel chiral preparation process of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one which is an inhibitor of ERK kinases (ERK1 and ERK2).

ERK protein belongs to the RAS/RAF/MEK/ERK pathway which plays a major role in cell cycle, proliferation, growth, and survival. RAS/RAF/MEK/ERK pathway is activated by growth factors through their receptor tyrosine kinase that allows activation of GTPases RAS. In its turn, RAS activates RAF proteins. Then, RAF activates MEK, which activates ERK.

Finally, this enables phosphorylation of many substrates that have key roles in metabolism, protein synthesis, cell proliferation and survival.

RAF mutations lead specifically to an over-activation of this RAS/RAF/MEK/ERK pathway and are responsible for 7% of all human cancers (Davies et al., Nature. 2002*;* Garnett et al., Cancer Cell. 2004).

Indeed, RAF mutations are frequently observed in melanomas (27-70%), thyroid cancers (36-53%), colorectal cancers (5-22%) and ovarian cancers (30%). Likewise, RAS mutations occur in almost 30% of cancers and are present in pancreatic (90%), lung (35%), colorectal (45%) and liver (30%) cancers (Downward, Nat. Rev. Cancer. 2003).

Thus, proteins of RAS/RAF/MEK/ERK pathway represent targets of interest for cancers treatment. Indeed, pharmaceutical companies are focusing on upstream kinases (RAF, MEK).

However, resistances ultimately appear after current treatment with RAF and MEK inhibitors (Lito et al., Nat. Med. 2013*;* Caunt et al., Nat. Rev. Cancer, 2015).

Moreover, most resistances to MEK or RAF inhibitors induce ERK reactivation, through different mechanisms such as MEK mutation, B-RAF amplification, C-RAF mutation... *(*Little et al., Oncogene. 2013*).*

Furthermore, RAF or MEK inhibition suppresses ERK negative feedback that restores upward signaling and finally ERK activity (Lito et al., Nat. Med., 2013).

Considering the resistance phenomena that emerged after current treatment with RAF and MEK inhibitors, it is essential to develop new therapeutic options.

Except for its key role in hyperproliferative diseases, ERK signaling has also been described as implied in neurodegenerative disorders such as in Parkinson's, Alzheimer's and Huntington's diseases (Cheung et al., Sci. STKE. 2004*;* Bodai et al., Bioessays., 2012) and in inflammation such as in the pathogenesis of Rheumatoid Arthritis (Thalhamer et al., Rheumatology. 2008).

The compound (*S*)-1-(1-(3-chlorophenyl)-2-(dimethylamino)ethyl)-4-(5-morpholino-1*H-*pyrrolo[2,3-*b*]pyridin-3-yl)pyridin-2(1*H*)-one of formula (I), depicted below, has been developed, and is an efficient inhibitor of ERK kinases (ERK1 and ERK2). It may be used in particular as anticancer agent.

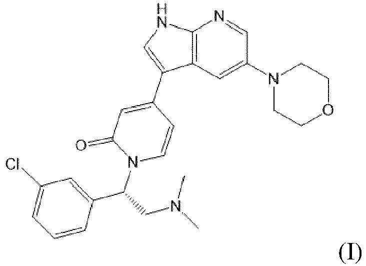

A racemic synthesis of this compound has already been described in WO2023135233.

An important disadvantage of known synthesis is thus that it leads to a racemic mixture. Thus, such synthesis requires a final chiral separation. Therefore, half of the materials prepared along the steps goes to waste.

Consequently, the yield of the known process is not high enough.

Further, while feasible on small scale for the initial in vitro and animal research phase, the known process poses the problems of scalability in terms of time, cost and general applicability to clinical development phase for industrial preparation of a pharmaceutically active ingredient.

There is therefore a need to provide new means for improving the preparation of (S)-1-(1-(3-chlorophenyl)-2-(dimethylamino)ethyl)-4-(5-morpholino-1*H*-pyrrolo[2,3-*b*]pyridin-3-yl)pyridin-2(1*H*)-one, and to develop a chiral process for the preparation of this compound.

The present invention is precisely directed to a novel chiral process of preparing (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one.

Thus, a first subject of the invention concerns a process for the preparation of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one comprising performing a Sharpless asymmetric dihydroxylation on a compound of formula (2): 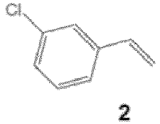 to selectively form a chiral compound of formula (3): 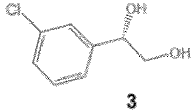 and then converting said compound of formula (3) into the corresponding chiral epoxide of formula (4):

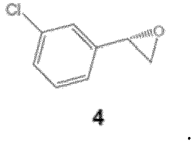

Another subject of the invention concerns a process for the preparation of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one comprising a Suzuki coupling reaction of a compound of formula (7) and a compound of formula (11):

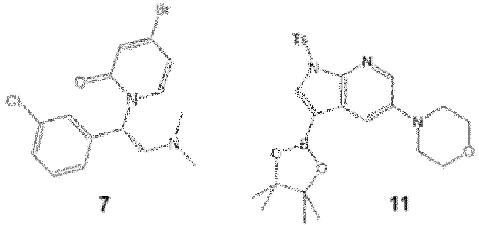

The present invention also relates to a process for the preparation of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one, characterized in that:
A) a compound of formula (1): 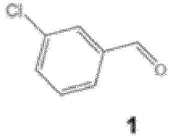 is subjected to a direct olefination to obtain a compound of formula (2): 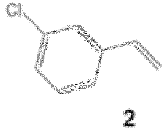
B) the compound of formula (2) is subjected to a Sharpless asymmetric dihydroxylation to form the chiral compound of formula (3): 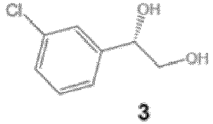
C) the compound of formula (3) is converted to the corresponding chiral epoxide of formula (4): 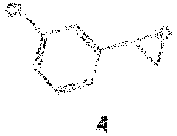
D) the compound of formula (4) is then put in the presence of dimethylamine, to form a chiral compound of formula (5): 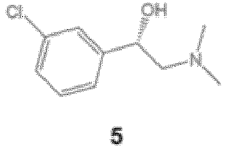
E) the compound of formula (5) is converted to the chiral compound of formula (6): 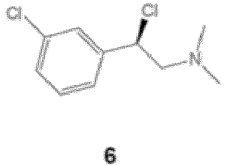
F) the compound of formula (6) is subjected to a reaction of *N*-alkylation with 4-bromo-1*H*-pyridin-2-one to give the chiral compound of formula (7): 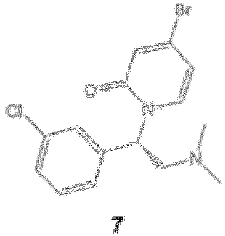
G) a compound of formula (8): 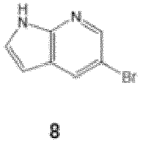 is subjected to a Buchwald-Hartwig coupling reaction with morpholine to obtain a compound of formula (9): 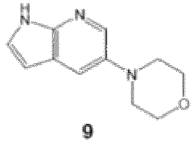
H) the compound of formula (9) is subjected to a protection reaction of the pyrrolyl moiety of the 7-azindole core to obtain a compound of formula (10): 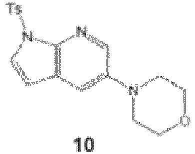
I) the compound of formula (10) is subjected to a selective borylation of the azaindole core in position 3 to obtain a compound of formula (11): 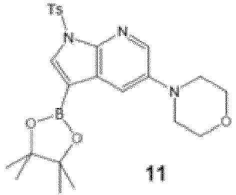
J) the compound of formula (7) and the compound of formula (11) are subjected to a Suzuki coupling reaction to give the chiral compound of formula (12): 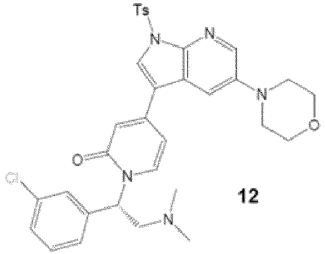 and
K) the compound of formula (12) is subjected to a deprotecting reaction to give (5)-1-(1-(3-chlorophenyl)-2-(dimethylamino)ethyl)-4-(5-morpholino-1*H*-pyrrolo[2,3-*b*]pyridin-3-yl)pyridin-2(1*H*)-one.

The process of the present invention presents the advantages of selectively preparing (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one, without requiring a final chiral separation.

In addition, the process provides high yielding synthesis of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one, as well as robust and scalable conditions, which fulfills the current clinical and industrial requirements.

### ABBREVIATIONS AND DEFINITIONS

In the context of the present invention, the following abbreviations and empirical formulae are used:
- CaCl₂: Calcium Chloride
- CDCl₃: Deuterated chloroform
- CH₃CN: Acetonitrile
- Cs₂CO₃: Caesium carbonate
- DCM: Dichloromethane
- (DHQ)₂PHAL: Hydroquinine 1,4-phthalazinediyl diether
- DMA: Dimethylamine
- DMF: Dimethylformamide
- DMSO: Dimethylsulfoxide
- °C: Degree Celsius
- Eq: Equivalent
- Et₂O: Diethyl ether
- EtsN: Triethylamine
- EtOAc: Ethyl acetate
- EtOH: Ethanol
- g: gram(s)
- h: hour(s)
- H₂CO₂: Formic acid
- HPLC: High performance liquid chromatography
- IR: Infrared Spectroscopy
- K₂CO₃: Potassium carbonate
- Kg: Kilogram
- L: Liter
- LC/MS: Liquid chromatography/mass spectrometry
- LiHMDS: Lithium bis(trimethylsilyl)amide
- M: Mole(s) per liter
- MeCN: Acetonitrile
- MeOH: Methanol
- MeONa: Sodium methoxide
- mg: Milligram(s)
- MH+: Pseudo-molecular ion (positive ion mode in mass spectrometry)
- MS: Mass Spectrometry
- µl: Microliter(s)
- ml: Milliliter(s)
- mmol: Millimole(s)
- mol: Mole(s)
- MsCl: Mesyl chloride
- Na₂CO₃: Sodium carbonate
- NaH: Sodium hydride
- NaHCO₃: Sodium hydrogen carbonate
- Na₂CO₃: Sodium carbonate
- Na₂SO₄: Sodium sulfate
- NH₄Cl: Ammonium chloride
- NMR: Nuclear Magnetic Resonance
- Por: Porosity
- RH: Relative Humidity
- RuPhos: 2-Dicyclohexylphosphino-2',6'-diisopropoxybiphenyl
- RuPhos Pd G2: Chloro(2-dicyclohexylphosphino-2',6'-diisopropoxy-1,1'-biphenyl)[2-(2'-amino-1,1'-biphenyl)]palladium(II)
- RuPhos Pd G3: (2-Dicyclohexylphosphino-2',6'-diisopropoxy-1,1'biphenyl)[2-(2'-amino-1,1'-biphenyl)]palladium(II) methanesulfonate
- RuPhos Pd G4: [Dicyclohexyl(2',6'-diisopropoxy-2-biphenylyl)phosphine-κ*P*](methanesulfonatato-κ*O*)[2'-(methylamino-κ*N*)-2-biphenylyl-κ*C*²]palladium
- TBAF: Tetrabutylammonium fluoride
- tBuOH: tert-Butyl alcohol
- tBuOK: Potassium tert-butoxide
- TEBAC: Benzyltriethylammonium chloride
- TEOA: Triethyl orthoacetate
- THF: Tetrahydrofuran
- TLC: Thin Layer Chromatography
- TMSCl: Trimethylsilyl chloride
- Ts: Tosyl
- UV: Ultraviolet

As used herein, the term "ambient temperature" or "room temperature" refers to a temperature ranging from 15°C to 30°C, more particularly from 18°C to 25°C.

Other features, properties and advantages of the invention will emerge more clearly from the description and examples that follow.

### DETAILED DESCRIPTION

### Olefination

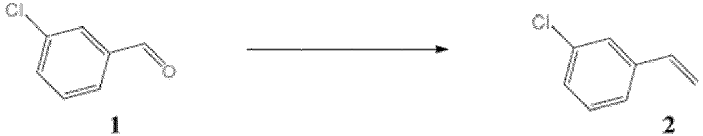

The compound of formula (2) is obtained by direct olefination of commercially available aldehyde of formula (1).

The reaction is generally performed with methyltriphenylphosphonium bromide in presence of a base, such as NaH or K₂CO₃, preferably K₂CO₃, and in an anhydrous solvent, such as 1,4-dioxane, Et₂O, DCM or THF, preferably 1,4-dioxane.

In particular, the reaction is performed at a temperature varying from 90°C to 110°C, preferably at 100°C.

In particular, the reaction is performed for 1 to 3 hours, in particular for 1 to 2 hours, preferably for 1.5 hours.

In particular, the reaction is performed under argon atmosphere.

### Sharpless asymmetric dihydroxylation

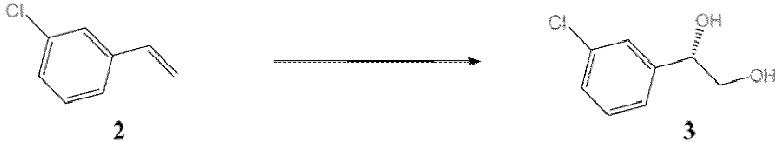

The compound of formula (3) is obtained by performing a Sharpless asymmetric dihydroxylation of the compound of formula (2).

A Sharpless asymmetric dihydroxylation, also named a Sharpless bishydroxylation, is a chemical reaction of an alkene with osmium tetroxide in the presence of a chiral quinine ligand to form a vicinal diol.

In particular, the reaction of Sharpless asymmetric dihydroxylation is performed in presence of K₃Fe(CN)₆, K₂CO₃, K₂Os(OH)₄ and (DHQ)₂PHAL. This dihydroxylation can also be performed directly with AD-mix-α ασ ρεαχταντ.

The reaction is generally performed in presence of a solvent, in particular in tBuOH or in a mixture tBuOH/H₂O, and preferably the solvent is tBuOH.

In particular, the reaction is performed at room temperature.

In particular, the reaction is performed for 15 to 20 hours, preferably for 16 hours.

### Epoxidation of the compound of formula (3)

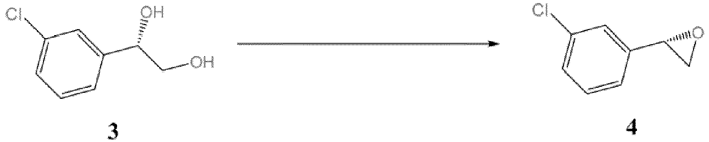

The compound of formula (4) is obtained by performing an epoxidation of the compound of formula (3).

In particular, the reaction is performed in two steps.

In particular, the first step is performed with the compound of formula (3), triethyl orthoacetate (TEOA) and trimethylsilyl chloride (TMSCl), in presence of a solvent, in particular DCM.

In particular, the first step is performed at 0°C.

In particular, the first step is performed for 2 to 4 hours, preferably for 3 hours.

In particular, the first step is performed under argon atmosphere.

In particular, the second step is performed with K₂CO₃, in presence of a solvent, in particular methanol.

In particular, the second step is performed at 0°C.

In particular, the second step performed for 0.1 to 2 hours, preferably for 1 hour.

In particular, the second step is performed under argon atmosphere.

In particular, the first step is performed in the presence of triethyl orthoacetate (TEOA) and trimethylsilyl chloride (TMSCl) and a solvent, and the second step is performed in the presence of K₂CO₃, and a solvent.

More particularly, the first step is performed in the presence of triethyl orthoacetate (TEOA) and trimethylsilyl chloride (TMSCl) and DCM as a solvent, and the second step is performed in the presence of K₂CO₃, and methanol as a solvent.

### Regioselective ring opening

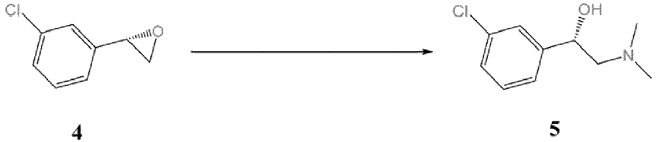

Selective epoxide opening of the compound of formula (4) is carrying out with commercially available dimethylamine.

The reaction is generally performed with a solvent, in particular in EtOH or in an EtOH/water mixture, preferably in EtOH, more preferably in EtOH 96%.

In particular, the reaction is performed at 0°C for 0.5 to 2 hours, preferably for 1 hour, and then at room temperature for 20 to 24 hours, preferably for 21 hours.

### Conversion of the alcohol into the corresponding chloroderivative

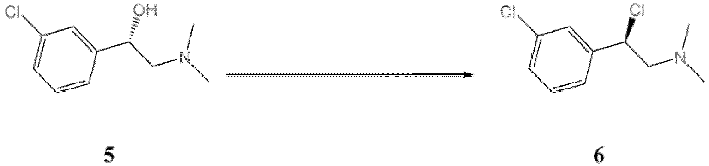

The benzylalcohol derivative of formula (5) is converted to the corresponding benzylchloride derivative of formula (6) using MsCl and EtsN.

The reaction is generally performed in presence of an anhydrous solvent, preferably DCM. In particular, the reaction is performed at 0°C.

In particular, the reaction is performed under argon atmosphere.

### N-alkylation

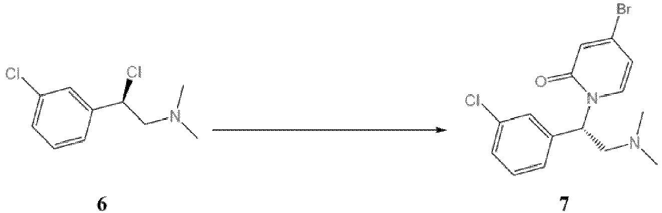

The compound of formula (7) is obtained by a substitution of the chlorine atom, using commercially available 4-bromo-1*H*-pyridin-2-one in the presence of a base such as K₂CO₃ or Na₂CO₃, preferably K₂CO₃, and in DMF or DMA, preferably in DMF.

The reaction could also be done in another solvent such as THF, Et₂O, DCM or acetone and with other bases like NaOH or tBuOK.

In particular, the reaction is performed at room temperature.

In particular, the reaction is performed for 10 to 20 hours, preferably for 14 hours.

### Buchwald-Hartwig coupling reaction

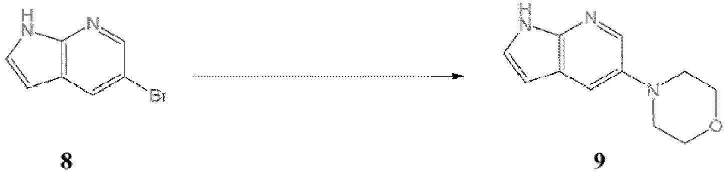

The 5-morpholino-7-azaindole compound of formula (9) is obtained from commercially available compound of formula (8), by carrying out a Buchwald-Hartwig coupling reaction in the presence of morpholine, with a base such as LiHMDS, a catalyst like RuPhos and a RuPhos ligand such as RuPhos Pd G2. Other RuPhos ligands such as RuPhos Pd G3 or RuPhos Pd G4 could also be used to obtain compound of formula (9). Preferably, the reaction is performed with LiHMDS as a base, RuPhos as catalyst and RuPhos Pd G2 as ligand.

This reaction is generally performed in an anhydrous solvent, preferably THF.

In particular, the reaction is performed at a temperature varying from 60°C to 70°C, preferably at 66°C.

In particular, the reaction is performed for 1 to 3 hours, in particular for 1 to 2 hours, preferably for 1.5 hours.

In particular, the reaction is performed under argon atmosphere.

### Protection of the pyrrolyl moiety of the 7-azindole core

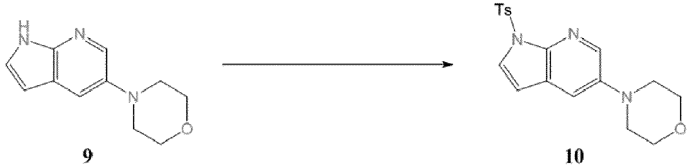

The compound of formula (10) is obtained by performing a protection of the pyrrolyl moiety of the 7-azaindole core with Ts as a protecting group, and using TEBAC, a base, such as NaOH or K₂CO₃, preferably NaOH, in an anhydrous solvent, such as DMF, DCM or THF, preferably DCM.

This reaction is generally performed at 0°C to room temperature.

In particular, the reaction is performed for 3 to 5 hours, preferably for 4 hours.

### Selective borylation

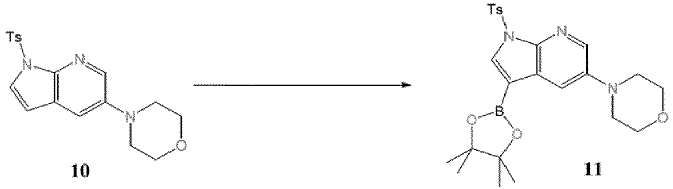

The compound of formula (11) is obtained by a selective borylation of the azaindole core in position 3, using (1,5-cyclooctadiene)(methoxy)iridium(I), in presence of a ligand, such as 4,4'-di-*tert*-butylbipyridine, and a source of boron, such as bis(pinacolato)diboron.

In particular, the reaction is performed in methyltetrahydrofuran.

In particular, the reaction is performed at reflux, preferably at 80°C, for 30 min to several hours, preferably for 1 hour.

In particular, the reaction is performed under argon atmosphere.

### Suzuki coupling reaction

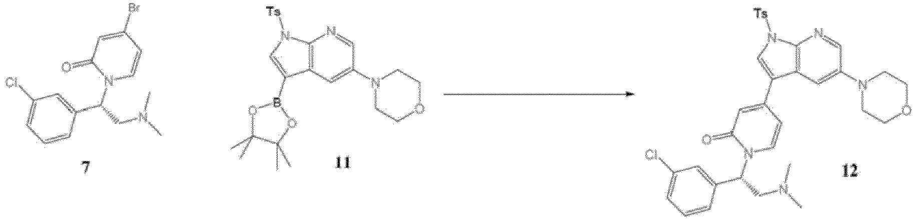

The compound of formula (12) is prepared by coupling synthetic intermediates of formula (7) and (11).

In particular, the Suzuki coupling reaction is performed in the presence of a base, like K₂CO₃ or Na₂CO₃ (in powder or in aqueous solution), preferably Na₂CO₃, and a palladium II catalyst, preferably bis(triphenylphosphine)palladium dichloride, in MeCN, in particular at a temperature comprised between 60°C and 110°C.

Other conditions can be used for this step, for example other solvents, such as 1,4-dioxane, DMSO, 1,2-dimethoxyethane, EtOH or DMF, other catalysts, such as palladium chloride or tris(dibenzylideneacetone)dipalladium, or other bases, such as NaOH or Cs₂CO₃.

In particular, the reaction is performed at a temperature varying from 60°C to 110°C, preferably at 70°C.

### Deprotection of the protecting group

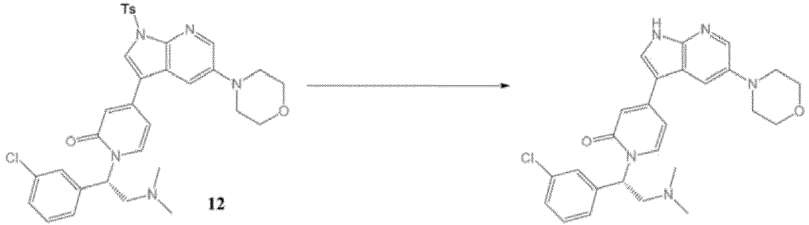

The compound of formula (12) is then deprotected to give (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one by using NaOH in DMSO at room temperature or Na₂CO₃ solution at 110°C.

This step could also be performed with other conditions like TBAF in THF at 66°C, or MeONa in MeOH at room temperature.

Preferably, this step is performed by using NaOH in DMSO at room temperature.

In particular, the reaction is performed for 2 to 4 hours, preferably for 3 hours.

According to a particular embodiment, the process of the present invention may be accomplished according to the Schemes 1 to 3 below.

In particular, the synthetic intermediate of formula (7) may be prepared according to Scheme 1 below.

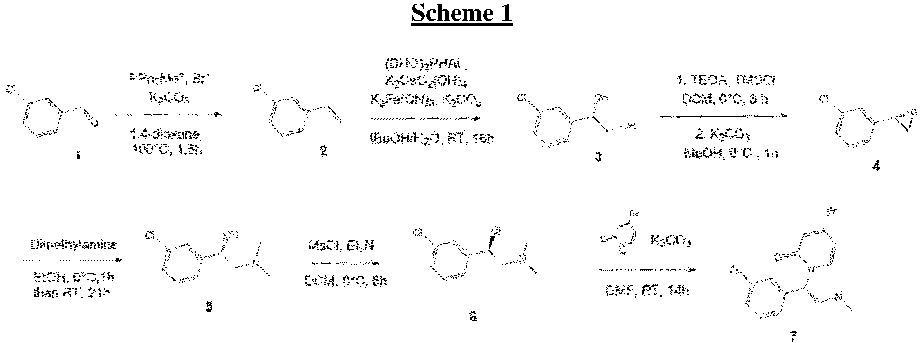

The compound of formula (2) is obtained by direct olefination of commercially available aldehyde of formula (1). The reaction is generally performed with methyltriphenylphosphonium bromide in presence of a base, such as K₂CO₃, and in a solvent such as 1,4-dioxane. The reaction may be performed at 100°C for 1 to 2 hours, in particular for 1.5 hours.

Alkene of formula (2) is converted to the chiral compound of formula (3), using a solvent such as tBuOH/H₂O, and using K₃Fe(CN)₆, K₂CO₃, K₂Os(OH)₄ and (DHQ)₂PHAL. The reaction may be performed at room temperature, for example for 16 hours.

Then, the compound of formula (3) is converted to the corresponding chiral epoxide of formula (4) using in a first step triethyl orthoacetate (TEOA) and trimethylsilyl chloride (TMSCl), and DCM as solvent, at 0°C, for 3 hours, and in a second step K₂CO₃ and methanol, at 0°C for 1 hour.

The chiral epoxide of formula (4) is then put in the presence of dimethylamine. The reaction is preferably performed in EtOH at 0°C for 1 hour, and then at room temperature for 21 hours.

The chiral benzylalcohol derivative of formula (5) is then converted to the corresponding chiral compound of formula (6) using MsCl and EtsN at 0°C in DCM as an anhydrous solvent.

The compound of formula (7) is finally obtained from the compound of formula (6) using commercially available 4-bromo-1*H*-pyridin-2-one in the presence of K₂CO₃ as a base, in DMF. The reaction may be performed at room temperature for 14 hours.

The present invention also concerns a process for preparing the compound of formula (7), wherein the following steps are carried out in that order, starting from the commercially available compound of formula (1):
1) an olefination of the aldehyde to give the corresponding alkene of formula (2),
2) a conversion of the alkene to a vicinal diol to obtain the chiral compound of formula (3), by performing a Sharpless asymmetric dihydroxylation,
3) an epoxidation to obtain the chiral compound of formula (4),
4) a regioselective ring opening to conduct to the chiral benzylalcohol of formula (5),
5) a conversion of the chiral alcohol into the corresponding chiral chloroderivative to give the compound of formula (6), directly followed by,
6) a *N*-alkylation of commercially available 4-bromo-1*H*-pyridin-2-one by the compound of formula (6) to finally give the compound of formula (7).

The synthetic intermediate of formula (11) may be prepared according to Scheme 2 as defined below.

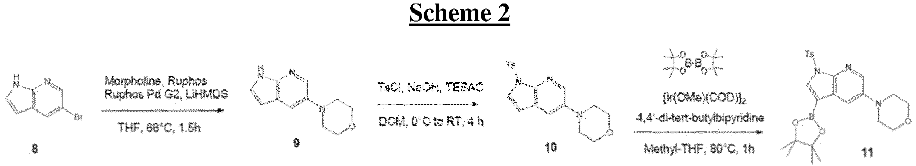

The 5-morpholino-7-azaindole compound of formula (9), as shown in Scheme 2, is obtained from the commercially available compound of formula (8), by carrying out a Buchwald-Hartwig coupling reaction in the presence of morpholine, with LiHMDS as base, RuPhos as catalyst and RuPhos Pd G2 as ligand. This reaction is generally performed in THF as anhydrous solvent and at a temperature of 60-70°C, for 1.5 hours.

Then, the compound of formula (10) is obtained by performing a protection of the pyrrolyl moiety of the 7-azaindole core with Ts as protecting group, and using NaOH as base, and TEBAC, in DCM as anhydrous solvent. This reaction is generally performed at 0°C to room temperature, for 4 hours.

Finally, the compound of formula (11) is obtained by a selective borylation of the azaindole core in position 3, using (1,5-cyclooctadiene)(methoxy)iridium(I), in presence of 4,4'-di-tert-butylbipyridine as ligand, and bis(pinacolato)diboron as a source of boron. This reaction is generally performed in methyltetrahydrofuran at 80°C for 1 hour.

Thus, the present invention also describes a process for preparing the compound of formula (11), wherein the following steps are carried out in that order, starting from the compound of formula (8):
a) a Buchwald-Hartwig coupling reaction to obtain the 5-morpholino-7-azaindole of formula (9),
b) a protection of the pyrrolyl moiety of the 7-azindole core to obtain the compound of formula (10), and
c) a selective borylation of the azaindole core in position 3 to finally obtain the compound of formula (11).

Finally, (*S*)-1-(1-(3-chlorophenyl)-2-(dimethylamino)ethyl)-4-(5-morpholino-1*H*-pyrrolo [2,3-*b*]pyridin-3-yl)pyridin-2(1*H*)-one may be prepared according to Scheme 3, as defined below.

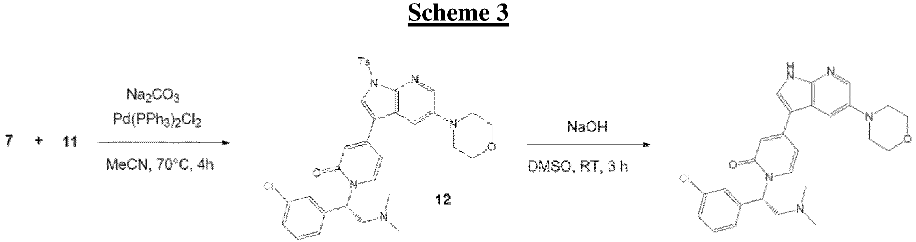

The compound of formula (12) is prepared by coupling synthetic intermediates of formula (7) and (11) previously described in Schemes 1 and 2.

The Suzuki coupling reaction is typically performed in the presence of a base like Na₂CO₃ (in powder or in aqueous solution), a palladium II catalyst such as bis(triphenylphosphine)palladium dichloride in MeCN, at a temperature comprised between 60°C and 110°C, preferably at 70°C.

The compound of formula (12) can be then deprotected to give (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one by using NaOH in DMSO at room temperature, for 3 hours.

According to an embodiment, (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one may be prepared by a process wherein the following steps are carried out in that order, starting from synthetic intermediates of formula (7) and (11):
i. a Suzuki coupling reaction performed at a temperature comprised between 60°C and 110°C, to give the compound of formula (12), and
ii. a deprotection of the protecting groups to give (*S*)-1-(1-(3-chlorophenyl)-2-(dimethylamino-1*H*-pyrrolo[2,3-*b*]pyridin-3-yl)pyridin-2(1*H*)-one.

The present invention will be better understood by referring to the following examples which are provided for illustrative purpose only and should not be interpreted as limiting in any manner the instant invention.

### EXAMPLES

### Equipment and analytical methods used for the syntheses of examples

### Flash chromatography:

Apparatus: Biotage SP with auto-collector and UV detection (2 wavelengths).
Normal phase columns: 120 g or 300 g Biotage external dry load cartridge kit, packed with Sigma-Aldrich 40-63 µm silica gel.

### Liquid Chromatography:

Apparatus: Waters alliance 2695 HPLC system with autosampler and Waters 2996 diode array detector.
Column: Macherey-Nagel Nucleoshell RP18 plus (5 µm, 4 mm x 100 mm).
Column temperature: 40°C.
Solvents: A (H₂O 99.9%, H₂CO₂ 0.1%); B (CH₃CN 99.9%, H₂CO₂ 0.1%).
Flow rate: 1 mL/min.
Gradient (A/B v/v): 90/10 (t = 0 min), 90/10 (t = 1 min), 0/100 (t = 7 min), 0/100 (t = 10 min).
Detection: 210-400 nm range.

### Chiral Chromatography:

Chiral column: Daicel ChiralPak IG (Amylose-based) 20 µm, 4.6 mm x 100 mm.
Chiral column: Daicel ChiralPak IG (Amylose-based) 5 µm, 4.6 mm x 250 mm.
Column temperature: 25°C.
Analysis of compound 3 (Isocratic conditions): Solvents Heptane 95% / EtOH containing 0.1% EtsN 5%, flow rate: 1mL/min.
Analysis of compound 7 (Isocratic conditions): Solvents Heptane 90% / EtOH containing 0.1% EtsN 10%, flow rate: 1mL/min.
Analysis of compound 12 (Isocratic conditions): Solvents Heptane 41.5% / EtOH containing 0.1% EtsN 32.5% and DCM 25%, flow rate: 1mL/min.
Analysis of final compound (*S*)-1-(1-(3-Chlorophenyl)-2-(dimethylamino)ethyl)-4-(5-morpholino-1*H*-pyrrolo[2,3-*b*]pyridin-3-yl)pyridin-2(1*H*)-one (Isocratic conditions): Solvents Heptane 50% / EtOH containing 0.1% EtsN 40% and DCM 10%, flow rate: 1mL/min.

### Mass Spectrometer:

Apparatus: Waters Micromass ZQ (simple quad).
Mass detection method: Electrospray positive mode (ESI+), mass range: 50-800 uma.

### NMR Spectrometer:

Apparatus: Bruker 400 MHz.
Methods: ¹H NMR spectra performed in DMSO-d6 using DMSO-d5 as internal reference, chemical shifts expressed in parts per million (ppm), signals expressed as follows: singlet = s, d = doublet, t = triplet, q = quadruplet, sept = septuplet, dd = double doublet, dt = double triplet, m = multiplet or large singlet, br = broad, H = proton.
³¹P NMR spectra performed in DMSO-d6, chemical shifts expressed in parts per million (ppm), signals expressed as follows: s = singlet.

### Example 1: Synthesis of (S)-1-(1-(3-Chloronhenyl)-2-(dimethylamino)ethyl)-4-(5-morpholino-1H-pyrrolo[2,3-b]pyridin-3-yl)pyridin-2(1H)-one

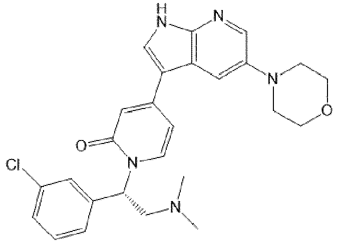

### Step 1: 3-Chlorostyrene

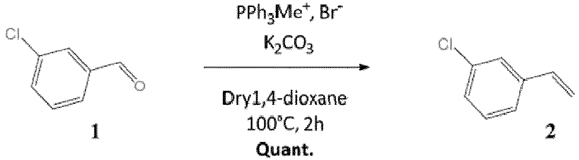

150 g of aldehyde **1** (1.07 mol) are added to a solution that contained the triphenyl phosphonium ylide reactive (457.4 g, 1.28 mol) dissolved in 900 ml of anhydrous 1,4-dioxane under Argon atmosphere. The white suspension is then heated at reflux for 2 hours. The reaction is then cooled to room temperature and evaporated under reduced pressure. The residue is diluted with 1,2 L of Et₂O and stirred for 45 min. The crude is then filtrated in a funnel (por 3) charged with celite (20 cm x 4 cm), previously washed with Et₂O. The solid is rinsed 3 times with 0.2 L of Et₂O. The filtrate is evaporated and the residue is redissolved in 200 mL of pentane. The pentane solution is filtrated over two silica pad (200 g SiO₂) and then eluted with 800 ml of pentane, followed by 0.2 L of pentane/Et₂O 95/5 to give 96.7 g of the compound **2** as a yellow oil.
Yield: Quantitative.
TLC: Rf = 0.2 (solvent 3% Et₂O/Heptane).
LC purity: 90%.
MH+: non ionizable.

### Step 2: 1-(3-Chlorophenyl)ethane-1,2-diol

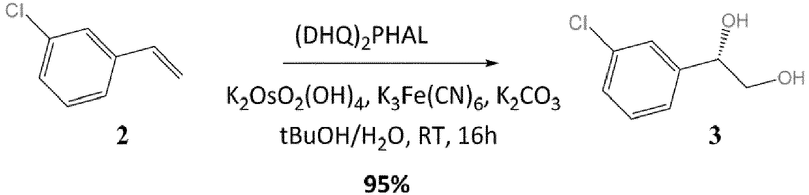

In three different flasks of 5 L placed at 0°C in an ice bath, 4.3 L of water are added (third volume on each flask). K₃Fe(CN)₆ (351 g, 1.07 mol), K₂CO₃ (148 g, 1.07 mol), K₂Os(OH)₄ (65 mg, 178 µmol), (DHQ)₂PHAL (2.77 g, 3.57 mmol) are added in each flask, followed by 49.3 g of 3-chlorostyrene **2** (described in the previous step) (0.36 mol) dissolved in 1.43 L of tBuOH (temperature maintained below 15°C during the addition). The different mixtures are stirred at 0°C for 1 h, then allowed to slowly warm up to reach room temperature and stirred for 16 hours. 1.2 L of EtOAc are then added into each reaction and stirred for 15 min at room temperature. Each flask precipitate is filtered on celite and rinsed with EtOAc (2 x 800 ml). Filtrates are combined, decanted and aqueous layer separated. Organic layer is washed with water/brine (3/1) (for each 1 L of organic phase, 200 ml of water/brine are used). All organic layers are washed by 2 x 300 ml of brine. Organic layers are combined and dried over Na₂SO₄, filtered and evaporated to dryness to give a residue of more of 217.3 g as a black oil. Purification is performed by filtration on silica pads divided in two batches (200 g, limited by funnel size) and dissolving the crude with 5% EtOAc/Hexane. The oil is dragged with the eluent for each step of elution. Product elution with 1.5 L of 5% EtOAc/Hexane per batch and then 50% EtOAc/Hexane until no more product was then eluted ~ 2 L (control TLC) to obtain 174.2 g of diol **3** as a brown oil.
Yield: 95%.
TLC: Rf = 0.35 (solvent 50% EtOAc in Hexane).
LC purity: 78%.
Chiral purity: 98%.
MH+: 155.2 (M-OH)+.
¹H NMR (DMSO-d6, 600 MHz): δ 7.39-7.26 (m, 4H); 5.37 (d, *J*=4.5Hz, 1H); 4.75 (t, *J*=6.1Hz, 1H); 4.58-4.53 (m, 1H); 3.50-3.41 (m, 2H).

### Step 3: (S)-2-(3-Chlorophenyl)oxirane

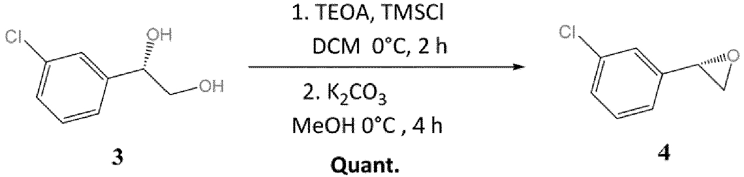

78.6 g of **diol 3** (455 mmol) are dissolved in 0.85 L of dry DCM and the solution is cooled down to 0°C under argon atmosphere. 104.3 ml of triethyl orthoacetate (TEOA, 570 mmol) diluted in 100 ml of DCM are then slowly added into the solution (temperature of reaction mixture at approx. 8°C, time of addition 45 min). 72.2 ml of trimethylsilyl chloride (TMSCl, 570 mmol) dissolved in 50 ml of DCM and cooled in an ice bath under argon atmosphere, are then slowly added with a dropping funnel and checking the temperature that was kept between 6 and 8°C (time of addition 45 min). The reaction is then stirred under argon atmosphere at 0°C for 30 min. 0.25 eq. of TEOA and 0.25 eq. of TMSCl are added and the reaction is kept stirring at 0°C for 1 hour more. The reaction is then evaporated (bath temperature 25°C) to give a pale yellow oil. The crude product is dissolved in dry methanol (470 ml) cooled at 0°C and 141.6 g of K₂CO₃ (1.02 mol) are slowly added keeping the temperature between 8 and 12°C (time of addition 15 min) under argon atmosphere. The reaction is stirred at 0°C for 3 hours. Reaction is warmed to 15°C for 1 hour. The reaction is filtrated on celite and concentrated under vacuum (bath temp 25°C). The cake is rinsed by 3 x 300 ml of Et₂O. The concentrated filtrate is dissolved in 600 ml of water and extracted with the Et₂O used to rinse the cake and with 2 x 300 ml of Et₂O more. Combined organic layers are washed with brine (600 ml), dried over Na₂SO₄, filtered and evaporated to dryness (bath temp 25°C) to give a pale yellow oil. Crude product (117.5 g) is diluted with 200 ml of pentane and filtered over a pad of silica (200 g, limited by funnel size) and rinsed with 1.5 L of pentane, 1.5 L of 10% Et₂O/pentane to obtain a pale yellow oil of 82.2 g of compound **4.**
Yield: Quantitative.
TLC: Rf = 0.3 (solvent 3% of Et₂O in pentane).
LC purity: 93.2%.
MH+: non-ionizable.
¹H NMR (DMSO-d6, 600 MHz): δ 7.41-7.32 (m, 3H); 7.28-7.25 (m, 1H); 3.97-3.95 (m, 1H); 3.14-3.10 (m, 1H); 2.88-2.85 (m, 1H).

### Step 4: (S)-1-(3-Chlorophenyl)-2-(dimethylamino)ethan-1-ol

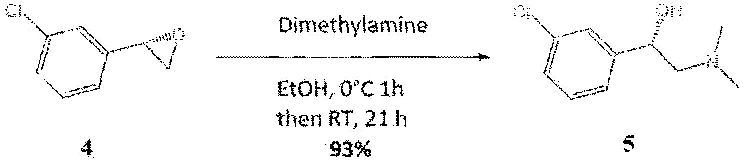

In a flask placed at 0°C in an ice bath, a solution of oxirane **4** (82.2 g, 531 mmol) in 1.3 L of ethanol 96% (v/v) is added followed by cold 532 ml of dimethylamine (2M solution in THF), checking temperature between 4 and 9°C. The solution is stirred for 1 hour at 0°C and then 21 hours at room temperature, and then it is stirred at 50°C for 2.5 hours. The crude is evaporated under vacuum and diluted in a mixture of 700 ml of EtOAc, 1 L of water and 300 ml of brine. The mixture is decanted and aqueous layer is extracted with EtOAc (300 ml × 2). Combined organic layers are dried over Na₂SO₄, filtered and evaporated to dryness to give 104.3 g of an orange-red liquid. The crude residue is diluted with 1.24 L of Et₂O and stirred at room temperature for 30 min. This solution is then filtrated on celite, rinsed by 2 x 150 ml of Et₂O and evaporated to dryness to give 99 g of desired compound 5 as a yellow oil.
Yield: 93%.
TLC: Rf = 0.28 (solvent 10% MeOH in DCM).
LC purity: 96.4%.
MH+: 200.3; 202.4 (M; M+2).
¹H NMR (DMSO-d6, 600 MHz): δ 7.40-7.25 (m, 4H); 5.16 (br s, 1H); 4.68-4.62 (m, 1H); 2.44-2.38 (m, 1H); 2.36-2.30 (m, 1H); 2.19 (s, 6H).

### Step 5: (R)-2-Chloro-2-(3-chlorophenyl)-N,N-dimethylethan-1-amine

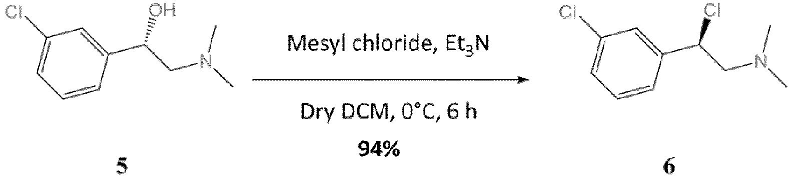

A solution of **5** (170 g, 851 mmol) in 1.7 L of dry DCM is cooled to 0°C in argon atmosphere and 356 ml of triethylamine (2.55 mol) are added. 132 ml of mesyl chloride (1.7 mol) are then added dropwise with a dropping funnel checking the temperature between 10 and 15°C (time of addition 1h40). The suspension is stirred at 0°C for 3h30 min. 0.2 equivalents of EtsN and mesyl chloride are added and stirred for 2 hours more. The reaction is quenched by the addition of 2 L of water and extracted using two funnels. Aqueous layer is then extracted with DCM (500 ml × 3). Combined organic layers are dried over Na₂SO₄, filtered and evaporated to dryness (bath at 20°C) to give 173.8 g of an orange sticky oil.
Yield: 94%.
TLC: Rf = 0.5 (solvent 10% MeOH in DCM).
MH+: 218.4; 220.4 (M; M+2).
¹H NMR (CDCl₃, 400 MHz): δ 7.41-7.39 (m, 1H); 7.32-7.27 (m, 3H); 4.92-4.86 m, 1H); 2.90-2.98 (m, 1H); 2.78-2.70 (m, 1H); 2.33 (s, 6H).

### Step 6: (S)-4-Bromo-1-(1-(3-chlorophenyl)-2-(dimethylamino)ethyl)pyridine-2(1H)-one

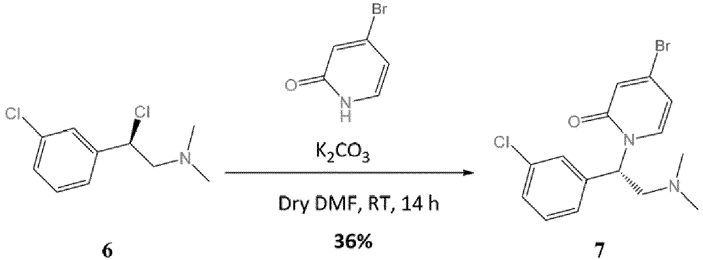

137 g of 4-bromo-1*H*-pyridin-2-one (787 mmol) are dissolved in 1.5 L of dry DMF to form a off-white suspension, then 130.6 g of K₂CO₃ (940 mmol) are added and the mixture is vigorously stirred for 1 hour at room temperature under argon atmosphere. Starting material **6** (171.8 g, 787 mmol) dissolved in 750 ml of dry DMF is then added and stirred for 14 hours at room temperature. The reaction mixture is then filtrated on celite, rinsed with Et₂O (2 x 300 ml) and the filtrate is divided into two flaks and concentrated until approx 150 ml. The residues are diluted with 700 ml of water into each flask and 300 ml of Et₂O and 300 ml of EtOAc are added. They are then triturated for 15 min and decanted. Aqueous layer is extracted by 2 x 300 ml of EtOAc and combined organic layers are washed with brine (600 ml), dried over Na₂SO₄, filtered and evaporated to dryness to give 203.6 g of a yellow solid. Purification is performed by flash chromatography on deactivated silica with triethylamine (four columns of 300 g) and the crude is loaded into the columns as a solid sample (the crude is dissolved in DCM and 400 g of deactivated silica are joined and highly dried under vacuum and divided into four for each column). Eluents used are hexane and EtOAc to obtain 103.9 g of the compound 7 as a pale yellow solid. A recrystallization is then performed: The residue is suspended in 300 ml of EtOAc and heated to reflux until we obtained a clear solution (final volume of 500 ml of EtOAc). 700 mL of hexane are then added in small portions. The solution is hot filtrated and the filtrate is cooled down to room temperature and then stored at 4°C overnight. The crystalline solid obtained is filtrated to yield 60.2 g of compound **7.**
Yield: 36%.
TLC: Rf = 0.3 (solvent 70 % EtOAc in DCM).
LC purity: 99.5%.
Chiral purity: 97.8%.
MH+: 355.4; 357.4; 359.5 (M; M+2; M+4).
¹H NMR (DMSO-d6, 600 MHz): δ 7.80 (d, *J*=7.5Hz, 1H); 7.44-7.41 (m, 1H); 7.40-7.35 (m, 2H); 7.31-7.26 (m, 1H); 6.73 (d, *J*=2.2Hz, 1H); 6.49 (dd, *J*=7.5 and 2.3Hz, 1H); 6.09-6.02 (m, 1H); 3.30-3.24 (m, 1H); 2.71-2.64 (m, 1H); 2.17 (s, 6H).

### Step 7: 4-(1H-Pyrrolo[2,3-b]pyridin-5-yl)morpholine

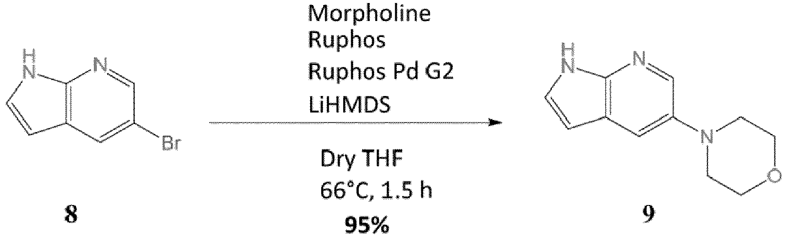

RuPhos (1.52 g, 3.25 mmol, 1% mol) and RuPhos PdG2 (2.52 g, 3.25 mmol, 1% mol) are dissolved in 780 ml of LiHMDS (1M in THF, 780 mmol) under argon atmosphere. 64 g of compound **8** (325 mmol) and 34 ml of morpholine (390 mmol) are then added and the reaction is stirred at reflux (66°C) for 1.5 hours. The solution is allowed to cool to room temperature and quenched by dropping into 1.9 L of an aqueous saturated NH₄Cl solution and stirred for 15 min. Aqueous layer is extracted with DCM (3 x 400 ml). Combined organic layers are dried over Na₂SO₄, filtered and evaporated to dryness to give 71 g of crude compound **9.** The residue is triturated in 300 ml of 30% EtOAc/hexane for 1 hour, then filtrated, rinsed with 300 ml of 10% EtOAc/hexane and dried under vacuum overnight to give rise 63 g of the compound **9** as a slightly brown thin powder.
Yield: 95%.
TLC: Rf = 0.37 (solvent EtOAc).
LC purity: 98.4%.
MH+: 204.3 (M+1).

### Step 8: 4-(1-Tosyl-1H-pyrrolo[2,3-b]pyridin-5-yl)morpholine

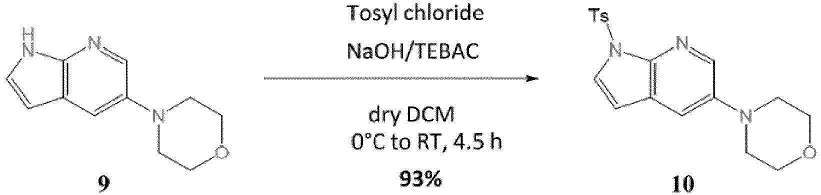

Pyrrolopyridine **9** (30 g, 147.6 mmol) is dissolved in anhydrous DCM (400 ml) and stirred at 0°C. Benzyltriethylammonium chloride, TEBAC (1 g, 4.43 mmol) and NaOH (17.71 g, 442.8 mmol) are then added. Tosyl chloride (33.77 g, 177.1 mmol) is then portion-wise added and stirred for 30 min more at 0°C. The reaction is allowed to warm to room temperature and stirred for 4 hours. 0.1 eq. of tosyl chloride is then added. The reaction is dropped into 2 L of fresh water and stirred for few minutes in an ice bath. It is then filtrated and washed with water. The solid precipitate is dissolved in DCM (1.5 L) and washed with NaHCO₃ (2 × 400 ml), water (2 × 400 ml) and brine (400 ml). Organic layer is then evaporated and the residue is triturated in 300 ml of 5% EtOAc/hexane for 2 hours. It is filtrated, rinsed with hexane and dried over P₂O₅ to give 48.93 g of the compound **10** as a pale brown powder.
Yield: 93%.
TLC: Rf = 0.41 (solvent 70% EtOAc in hexane).
LC purity: 99%.
MH+: 358.6 (M+1).
¹H NMR (DMSO-d6, 400 MHz): δ 8.18 (d, *J*=2.7Hz, 1H); 7.92 (d, *J*=8.3Hz, 2H); 7.78 (d, *J*=4.0Hz, 1H); 7.52 (d, *J*=2.7Hz, 1H); 7.39 (d, *J*=8.3Hz, 2H); 6.68 (d, *J*=4.0Hz, 1H); 3.76-3.71 (m, 4H); 43.12-3.07 (m, 4H); 2.33 (s, 3H).

### Step 9: 4-(3-(4,4,5,5-Tetramethyl-1,3,2-dioxaborolan-2-yl)-1-tosyl-1H-pyrrolo[2,3-b]pyridin-5-yl)morpholine

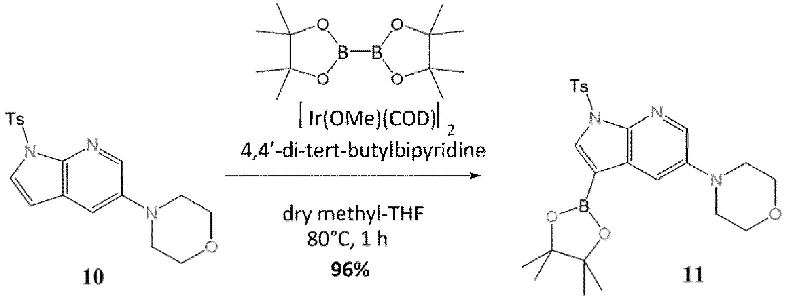

Pyrrolopyridine **10** (103.2 g, 289 mmol) is dissolved in 2-methyltetrahyrofuran under argon atmosphere and stirred at room temperature. Bis-pinacolato diborane (B₂pin₂, 80.7 g, 318 mmol), 4,4'-di-tert-butylbipyridine (3.1 g, 11.5 mmol) and (1,5-cyclooctadiene)(methoxy) iridium (I) dimer ([Ir(OMe)(COD)]₂, 3.8 g, 5.8 mmol) are then added. The reaction is heated to reflux for 50 min. Reaction is then cooled down to -10°C on an ice/acetone bath and is quenched by the addition of 600 ml of cold MeOH. First 12 ml are carefully and slowly added checking the temperature (during 30 min) and by then the rest of MeOH is added faster. Reaction is then stirred at room temperature for 15 min and evaporated to dryness to give a residue black/brown oil. The residue is dissolved in DCM (1.5 L) and washed with water (3 x 500 ml) and brine (500 ml). Organic layer is evaporated to give a 200 g of a black paste. 1.5 L of Et₂O are then added and stirred for 15 min at room temperature. The solution is then filtrated on a SiO₂ pad (1 Kg, with sand on the top) and the silica is rinsed with Et₂O (1.5 L × 3). Filtrate is evaporated and coevaporated with hexane to finally give a pale yellow solid foam, dried under vacuum over P₂O₅ to yield 146.5 g of the compound **11.**
Yield: 96%.
TLC: Rf = 0.5 (solvent 70% EtOAc in hexane).
LC purity: 90.8%.
MH+: 484.6 (M+1).
¹H NMR (DMSO-d6, 400 MHz): δ 8.21 (d, *J*=2.5Hz, 1H); 8.01 (d, *J*=8.3Hz, 2H); 7.94 (s, 1H); 7.50 (d, *J*=2.4Hz, 1H); 7.41 (d, *J*=8.0Hz, 2H); 3.78-3.72 (m, 4H); 3.12-3.07 (m, 4H); 2.33 (s, 3H); 1.30 (s, 12H).

### Step 10: (S)-1-(1-(3-Chlorophenyl)-2-(dimethylamino)ethyl)-4-(5-morpholino-1-tosyl-1H-pyrrolo[2,3-b]pyridin-3-yl)pyridin-2(1H)-one

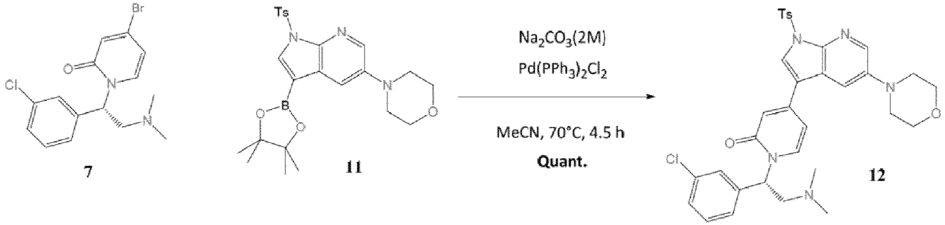

Reaction is divided in two 2 L flasks with half volume into each one. The bromopyridinone **7** (60.2 g, 169 mmol) and the compound **11** (98.2 g, 203 mmol) are dissolved in 903 ml of MeCN under argon atmosphere. Sodium carbonate 2M (903 ml) is added to give a biphasic mixture that is bubbled with argon for 15 min. Bis(triphenylphosphine)palladium (II) dichloride (Pd(PPh₃)₂Cl₂, 2.97 g, 4.2 mmol, 2.5 mol %) is then added and the solution is bubbled for another 15 min. The reaction is stirred at 70°C for 2 hours. 0.025 eq. of Pd(PPh₃)₂Cl₂ are then added and stirred for 1 hour. 0.025 eq more of Pd(PPh₃)₂Cl₂ are then added. 0.15 eq. of the compound **11** is then added and stirred for 0.5 hour. The reaction is allowed to cool down to reach room temperature. 1.4 L of cold water (700 ml for each one) are added and stirred in an ice bath for 60 min. An off-white solid crashes out between both layers which is filtrated through a sintered glass funnel (size 3). Solid is rinsed with cold deionised water (4 x 100 ml) and three times rapidly with Et₂O (4 x 150 ml) dried by suction filtration for at least 15 min and then dried in a desiccator over P₂O₅ to obtain 129 g of the compound **12** as a pale brown solid.
Yield: Quantitative.
TLC: Rf = 0,5 (solvent 70% EtOAc in hexane).
LC purity: 87%.
MH+: 633.0; 635.0 (M; M+2).
¹H NMR (DMSO-d6, MHz): δ 8.36 (s, 1H); 8.26 (d, *J*=2.5Hz, 1H); 7.99 (d, *J*=8.4Hz, 2H); 7.87 (d, *J*=7.3, 1H); 7.64 (d, *J*=2.6Hz, 1H); 7.49 (m, 1H); 7.44-7.33 (m, 5H); 6.80-6.72 (m, 2H); 6.22-6.14 (m, 1H); 3.77-3.71 (m, 4H); 3.20-3.14 (m, 4H); 2.76-2.67 (m, 1H); 2.33 (s, 3H); 2.20 (s, 6H).

### Step 11: (S)-1-(1-(3-Chlorophenyl)-2-(dimethylamino)ethyl)-4-(5-morpholino-1H-pyrrolo[2,3-b]pyridin-3-yl)pyridin-2(1H)-one

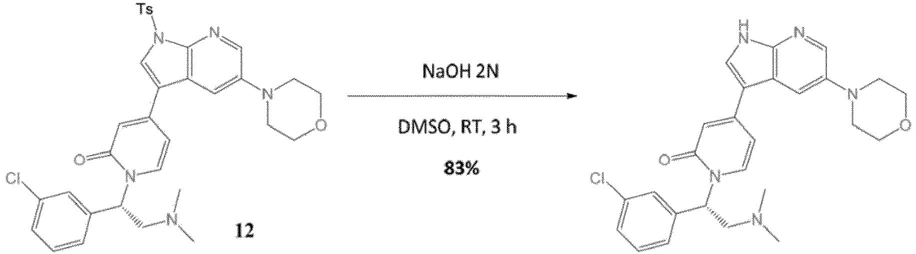

Reaction is divided in three equal batches. Compound **12** (107 g, 170 mmol) is suspended in 5 L of DMSO and cooled in an ice bath. Sodium hydroxide 2N solution (133 ml) is then slowly added keeping the temperature between 16 and 18°C. The reactions are then brought to room temperature and stirred for 1.5 hours. 0.4 eq of NaOH 2M are then added using the ice bath during the addition and stirred at room temperature for 1 hour more. The process is repeated adding 0.3 eq of NaOH until the reaction is finished (from 5 to 6.5 hours and 2.3 eq to 2.9 eq of NaOH in different batches). The reactions are then quenched by the addition of 0.42 L of NH₄Cl saturated solution into each reaction (volume total: 1.26L) slowly added and placed in an ice bath in order to keep the temperature below 20°C. The solutions are stirred for another 30 min, divided into two equal parts and each one is poured into 3.2 L of water (Volume total: 19.2 L). It is stirred for 1 hour in an ice bath and then the precipitate is filtrated through a sintered glass funnel (size 3). The yellow cake is washed with deionised water (4 × 200 ml) then with Et₂O (5 x 200 ml) and dried in a desiccator over P₂O₅ to obtain 86.2 g of (*S*)-1-(1-(3-Chlorophenyl)-2-(dimethylamino)ethyl)-4-(5-morpholino-1*H*-pyrrolo[2,3-*b*]pyridin-3-yl)pyridin-2(1*H*)-one as a pale yellow/brown solid. The product is triturated in 434 ml of EtOAc stirred at 60°C for 1 hour, cooled down to room temperature and filtrated. The product is then triturated again in 434 ml of EtOAc at 60°C. The solid is dried under vacuum for 2 hours and then triturated in 340 ml of Et₂O at room temperature for 2 hours, filtrated and dried over P₂O₅ overnight to obtain 67.5 g of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one as a very pale yellow powder.
Yield: 83%.
TLC: Rf = 0,25 (solvent 10% MeOH in DCM).
LC purity: 99.3%.
LC chiral: 98.6%.
MH+: 478.7; 480.7 (M; M+2).
¹H NMR (DMSO-d6, 400 MHz): δ 12.05 (br s, 1H); 8.17 (d, *J*=2.5Hz, 1H); 8.10 (d, *J*=2.9Hz, 1H); 7.78-7.74 (m, 1H); 7.71 (d, *J*=2.5Hz, 1H); 7.49-7.46 (m, 1H); 7.45-7.33 (m, 3H); 6.72-6.67 (m, 2H); 6.23-6.15 (m, 1H); 3.82-3.76 (m, 4H); 3.32-3.26 (m, 1H); 3.18-3.12 (m, 4H); 2.78-2.69 (m, 1H); 2.22 (s, 6H).

7 g of (*S*)-1-(1-(3-chlorophenyl)-2-(dimethylamino)ethyl)-4-(5-morpholino-1*H-*pyrrolo[2,3-*b*]pyridin-3-yl)pyridin-2(1*H*)-one are suspended in 1.2 L of solvent MeCN/H₂O (70/30). The solution is heated at reflux and several additions of solvent are performed until complete solubilization (total volume of 1.5 L). The solution is hot filtrated and the filtrate is then cooled down to room temperature and stored at 4°C overnight to give 4.1 g of pure (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one as off-white crystals.
TLC: Rf = 0.25 (solvent 10% MeOH in DCM)
LC purity: 100%
LC chiral: 99.5%
MH+: 478.7; 480.7 (M; M+2)

## Claims

1. A process for the preparation of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one comprising performing a Sharpless asymmetric dihydroxylation on a compound of formula (2): 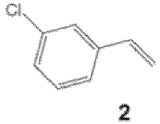 to selectively form a chiral compound of formula (3): 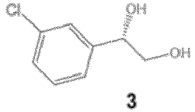 and then converting said compound of formula (3) into the corresponding chiral epoxide of formula (4):

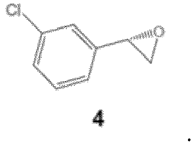

2. The process according to claim 1, wherein the reaction of Sharpless asymmetric dihydroxylation is performed in presence of K₃Fe(CN)₆, K₂CO₃, K₂Os(OH)₄ and (DHQ)₂PHAL.

3. The process according to any one of the preceding claims, wherein the reaction of Sharpless asymmetric dihydroxylation is performed in presence of a solvent.

4. The process according to the preceding claim, wherein the solvent is tBuOH.

5. The process according to any one of the preceding claims, wherein the reaction of Sharpless asymmetric dihydroxylation is performed at room temperature.

6. The process according to any one of the preceding claims, wherein the reaction of Sharpless asymmetric dihydroxylation is performed for 15 to 20 hours.

7. The process according to the preceding claim, wherein the reaction is performed for 16 hours.

8. The process according to any one of the preceding claims, wherein the reaction of epoxidation is performed in two steps.

9. The process according to the preceding claim, wherein the first step is performed in the presence of triethyl orthoacetate (TEOA) and trimethylsilyl chloride (TMSCl) and a solvent, and the second step is performed in the presence of K₂CO₃, and a solvent.

10. The process according to the preceding claim, wherein the first step is performed in the presence of triethyl orthoacetate (TEOA) and trimethylsilyl chloride (TMSCl) and DCM as a solvent, and the second step is performed in the presence of K₂CO₃, and methanol as a solvent.

11. A process for the preparation of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one comprising a Suzuki coupling reaction of a compound of formula (7) and a compound of formula (11):

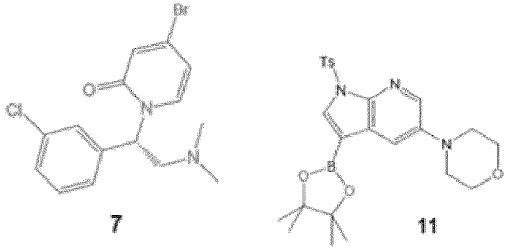

12. The process according to the preceding claim, wherein the reaction is performed in the presence of a base, preferably Na₂CO₃, and a palladium II catalyst, preferably bis(triphenylphosphine)palladium dichloride, in MeCN.

13. The process according to claim 11 or claim 12, wherein the reaction is performed at a temperature varying from 60°C to 110°C, preferably at 70°C.

14. A process for the preparation of (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one, **characterized in that**:
A) a compound of formula (1): 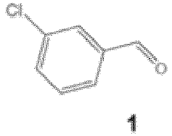 is subjected to a direct olefination to obtain a compound of formula (2): 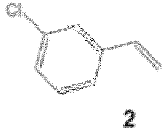
B) the compound of formula (2) is subjected to a Sharpless asymmetric dihydroxylation to form the chiral compound of formula (3): 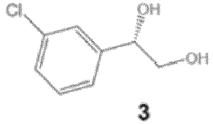
C) the compound of formula (3) is converted to the corresponding chiral epoxide of formula (4): 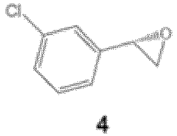
D) the compound of formula (4) is then put in the presence of dimethylamine, to form a chiral compound of formula (5): 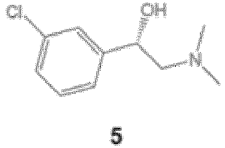
E) the compound of formula (5) is converted to the chiral compound of formula (6): 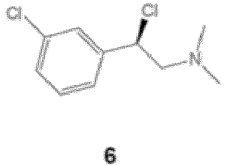
F) the compound of formula (6) is subjected to a reaction of *N*-alkylation with 4-bromo-1*H*-pyridin-2-one to give the chiral compound of formula (7): 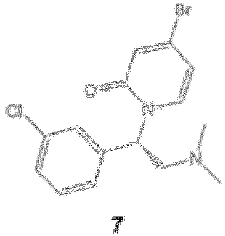
G) a compound of formula (8): 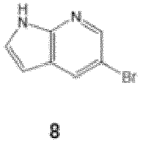 is subjected to a Buchwald-Hartwig coupling reaction with morpholine to obtain a compound of formula (9): 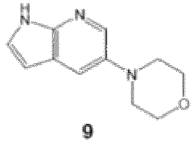
H) the compound of formula (9) is subjected to a protection reaction of the pyrrolyl moiety of the 7-azindole core to obtain a compound of formula (10): 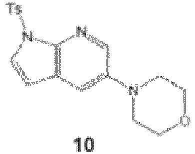
I) the compound of formula (10) is subjected to a selective borylation of the azaindole core in position 3 to obtain a compound of formula (11): 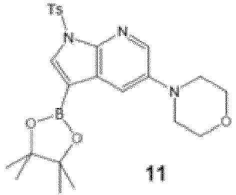
J) the compound of formula (7) and the compound of formula (11) are subjected to a Suzuki coupling reaction to give the chiral compound of formula (12): 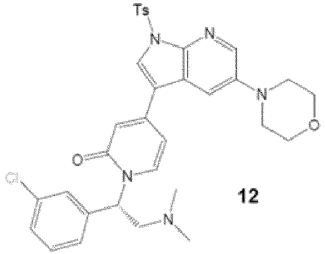 and
K) the compound of formula (12) is subjected to a deprotecting reaction to give (*S*)-1-(1-(3-chlorophenyl)-2-(dimethylamino)ethyl)-4-(5-morpholino-1*H*-pyrrolo[2,3-*b*]pyridin-3-yl)pyridin-2(1*H*)-one.
